# EUROPEAN PATENT SPECIFICATION

(11) **EP 2 094 308 B1**
(45) Date of publication and mention of the grant of the patent: **12.01.2011**
(21) Application number: 07853136.5
(22) Date of filing: 20.11.2007
(51) Int. Cl.: A61K 31/5513, A61K 47/40, A61K 9/00

(54) **STABLE PARENTERAL FORMULATION CONTAINING A RSV INHIBITOR OF A BENZODIAZEPINE STRUCTURE**
STABILE PARENTERALE FORMULIERUNG MIT EINEM RSV-HEMMER EINER BENZODIAZEPIN-STRUKTUR
FORMULATION PARENTÉRALE STABLE CONTENANT UN INHIBITEUR DE VSR D'UNE STRUCTURE DE BENZODIAZÉPINE

(30) Priority: 21.11.2006 US 866646 P
(43) Date of publication of application: 02.09.2009
(73) Proprietor: Novartis AG, 4056 Basel (CH)
(72) Inventor: BURANACHOKPAISAN, Thitiwan, Bedminster, New Jersey 07921 (US); JIANG, Wenlei, Germantown, Maryland 20874 (US); TONG, Wei-qin, Basking Ridge, New Jersey 07920 (US)
(74) Representative: von Sprecher, Georg
(86) International application number: PCT/US2007/024246
(87) International publication number: WO 2008/063634

(56) References cited:
- WO-A-2004/026843
- WO-A-2005/089769

## Description

The present invention relates to pharmaceutical formulations of benzodiazepine compounds which are active against Respiratory Syncytial Virus (RSV) suitable for parenteral administration.

RSV is a major cause of respiratory illness in patients of all ages. In adults, it tends to cause mild cold symptoms. In school-aged children, it can cause a cold and bronchial cough. In infants and toddlers it can cause bronchiolitis (inflammation of the smaller airways of the lungs), pneumonia, middle ear infections (otitis media) or lead to the development of asthma during childhood. RSV is the most common respiratory pathogen in infants and young children and numerous infants need to be hospitalized due to severe RSV disease, and about 1-2% of these infants die. Infants born prematurely, those with chronic lung disease, those who are immunocompromised, and those with certain forms of heart disease are at increased risk for severe RSV disease.

WO2004/026843 discloses certain benzodiazepine derivatives which are active against RSV. In order to appropriately treat RSV infections a parenteral route of administration is desireable. Accordingly, there is a need to develop parenteral formulations, and in particular formulations for intravenous administration, for benzodiazepine RSV inhibitor compounds. However, the poor aqueous solubility and stability of the benzodiazepine RSV inhibitor compounds described in WO2004/026843 and a high dose requirement for effective RSV treatment post a significant challenge for the development of parenteral pharmaceutical formulations of the benzodiazepine RSV inhibitor compounds.

It has now been found, in accordance with the present invention, that stable parenteral pharmaceutical formulations comprising a benzodiazepine RSV inhibitor compound and a beta-cyclodextrin, such as hydroxypropyl-beta-cyclodextrine, can be obtained.

### Summary of the Invention

The present invention provides a pharmaceutical composition suitable for parenteral administration comprising a benzodiazepine RSV inhibitor compound, or a pharmaceutically acceptable salt thereof, such as the besylate salt of (S)-1-(2-Fluoro-phenyl)-3-(2-oxo-5-phenyl-2,3-dihydro-1H-benzo[e][1,4]diazepin-3-yl)-urea, and a beta-cyclodextrin such as hydroxypropyl-beta-cyclodextrin (HPbCD) according to claims 1-3.WO2005089769 discloses pharmaceutical compositions comprising a) an inhibitor of the RSV fusion protein and b) a benzodiazepine derivative capable of inhibiting RSV replication. Liquid dispersions for oral administration, suspensions, emulsions, solutions for injection are proposed with a generalised list of possible excipients. Further there are provided uses for the manufacture of a medicament for treating a viral infection such as a RSV infection in a patient in need comprising parenterally administering a pharmaceutical formulation as disclosed above.

### Detailed Description of the Invention

The pharmaceutical composition according to the present invention is suitable for parenteral administration to humans, and in particular to infants and young children, for the treatment of viral infections.

The parenteral formulation of the present invention comprises a pharmaceutically effective amount of an benzodiazepine compound, or a pharmaceutical salt thereof, having the following structure (I): 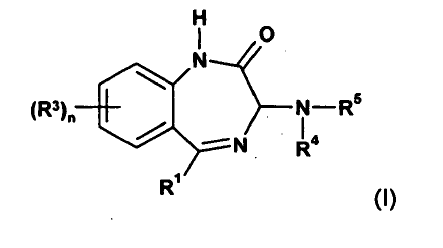 wherein the substituents are defined as described in WO04/026843, the contents of which are herewith incorporated by reference. The benzodiazepine compounds active against RSV described in WO04/026843 are termed hereinbelow as "benzodiazepine RSV inhibitor compounds."

A benzodiazepine RSV inhibitor compound of particular interest for the present invention is (S)-1 -(2-Fluoro-phenyl)-3-(2-oxo-5-phenyl-2,3-dihydro-1H-benzo[e][1,4]diazepin-3-yl)-urea which can also be described by the formula (Ia): 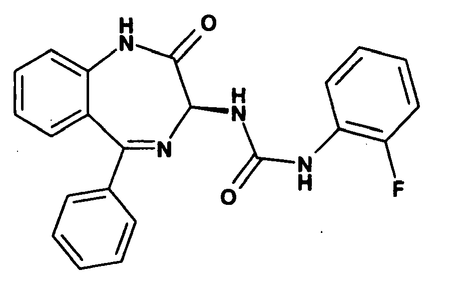 Other preferred RSV inhibitor compounds include (S)-4-Methanesulfonyl-2-methoxy-N-(2-oxo-5-phenyl-2,3-dihydro-1H-benzo[e][1,4]diazepin-3-yl)-benzamide or (S)-2-Chloro-4-morpholin-4-yl-N-(2-oxo-5-phenyl-2,3-dihydro-1H-benzo[e][1,4]diazepin-3-yl)-benzamide. In a preferred embodiment of the present invention, the RSV inhibitor compound is in the form of the besylate salt. The besylate salt of a compound of formula Ia may be in amorphous form or in a crystalline form as described in US patent application no. 60/802836. Synthesis routes for the benzodiazepine RSV inhibitor compounds are described for instance in WO2004/026843, the besylate salts and crystalline forms of(S)-1-(2-Fluorophenyl)-3-(2-oxo-5-phenyl-2,3-dihydro-1H-benzo[e][1,4]diazepin-3-yl)-urea can be prepared as described in US patent application no. 60/802836.

The term "pharmaceutically effective amount" as used herein indicates an amount necessary to administer to a host to achieve a therapeutic result, especially an inhibition of a viral infection such as e.g. a RSV infection. The terms "galenic formulation," "pharmaceutical formulation" or "formulation" as used herein refer to a pharmaceutical composition comprising the active ingredient and further excipients to make it suitable to apply to a patient and is usually a finished drug product. The term "formulation" and "pharmaceutical composition" may be interchangeably used herein depending on the context of these terms.

It has now been found in accordance with the present invention that parenteral formulations of the benzodiazepine RSV inhibitor compounds which comprise a beta-cyclodextrin compound have particularly advantageous properties with respect to e.g. stability and solubility. The term beta-cyclodextrin compound as used herein includes beta-cyclodextrin and, in particular, beta-cyclodextrin derivatives such as e.g. methyl-beta-cyclodextrin, dimethyl-beta-cyclodextrin, hydroxypropyl-beta-cyclodextrin, glycosyl-beta-cyclodextrin, maltosyl-beta-cyclodextrin, sulfonated-beta-cyclodextrin, sulfonated alkyl ether-beta-cyclodextrin (e.g. C₁₋₄ alkyl). Such formulations have been found suitable also for administration to infants and small children. Accordingly, the formulations of the present invention comprise at least a benzodiazepine RSV inhibitor and a beta-cyclodextrin compound which is preferably hydroxypropyl-beta-cyclodextrin (HPbCD), sulfobutyl ether beta-cyclodextrin (SBEbCD) or methyl beta-cyclodextrin (MbCD), most preferably HPbCD. HPbCD can for instance be prepared as described in US3'459'731 by propylene oxide addition to beta-cyclodextrin. HPbCD is also commercially available, e.g., from Cargill™ Pharmaceutical Exipients as Cavitron 82003, Cavitron 82004 or Cavitron (www.cargillexcipients.com). SBEbCD and MbCD are for instance commercially available from Shandong Xinda Fine Chemical Co., Ltd. The beta-cyclodextrin compound may be present in an amount, by weight, of an aqueous parenteral formulation of 10% to 60%, preferably 25% to 50% or 15% to 30%. In another preferred embodiment, the beta-cyclodextrin compound is present in an amount, by weight of at least 10%, at least 15% or at least 18% in the aqueous solution. In case the formulation is in form of a lyophilized cake, the beta-cyclodextrin compound is present in an amount of 50% to 99.9%, preferably 60% to 99.5%, more preferably 70% to 99%. Most conveniently the beta-cyclodextrin is present in an amount of between 90% to 99%, such as e.g. ca. 98%.

In a preferred embodiment, the parenteral formulation is suitable for intravenous administration. The immediate response of this form of administration is highly desirable in emergency situations, and in particular for infants and small children with RSV infection. Furthermore, as no absorption process is involved, the dose or blood concentration of active agent may be obtained with greater accuracy and speed. The term "infant" as used herein refers to children or babies from birth to about 2 years of age. The term "small child" as used herein refers to children from about to 2 to 10 years, preferably below 10, 8 or 6 years of age.

The parenteral formulations of the present invention can be for instance in form of an aqueous solutions. By "aqueous solutions" is meant a solution with the active ingredient, beta-cyclodextrine and optional pharmaceutical excipients was are dissolved using water as main solvent. The water may be buffered to stabilize the pH with a suitable buffer such as e.g. a phosphate buffer, acetate, citrate, lactate or maleate buffer. The buffer is preferably present in suitable amount to adjust the PH to the desired value, e.g. in a concentration of 10 mM to 100 mM. The "aqueous solution" may further contain a water-miscible organic solvent or solvents. When an organic co-solvent is employed it is preferred that the it is used in amounts of up to 10% by weight total solution, e.g. 0.5 to 10%. Suitable solvents are those water- miscible solvents commonly used in the art, for example propyleneglycol, polyethyleneglycol 300, polyethyleneglycol 400, glycerol, tween 20, tween 80 and ethanol.

The parenteral formulations suitable for intravenous administration are often formulated to have the approximately same osmotic pressure as body fluid such as e.g. blood. Accordingly, a parenteral formulation of the invention may comprises an isotonic agent which has the effect of rendering the osmotic pressure of the formulation the same as that of body fluid. In one aspect of the present invention there is provided a parenteral formulation comprising as active agent a benzodiazepine RSV inhibitor compound, e.g. (S)-1-(2-Fluorophenyl)-3-(2-oxo-5-phenyl-2,3-dihydro-1H-benzo[e][1,4]diazepin-3-yl)-urea, a beta-cyclodextrine, e.g., HPbC or SBEbCD, in a water-based or aqueous-based solution, a buffer and an isotonic agent. The isotonic agent may be selected from any of those commonly used in the art, e.g. sucrose, mannitol, trehalose, glycine, sodium chloride, dextran and glucose. The isotonic agents may be used in quantities which impart to the parenteral formulation the same osmotic pressure as body fluid. The precise amount necessary to achieve the desired effect may depend on factors such as the concentration of active agent in the parenteral formulation, and is a matter of routine experimentation which the skilled person may determine without exercising any inventive thought and using only common general knowledge. Selection of the isotonic agent is preferably made having regard to the properties, e.g. stability of the active agent.

The pH of the parenteral formulation of the present invention is typically maintained in the range of about 4 to 9, or more preferably in the range of about 6 to 8. In another embodiment, the pH is in a range of about 4 to about 6.

Parenteral formulations according to the invention may contain other excipients commonly employed in parenteral formulations suitable for intravenous administration in order to provide the required stability and therapeutic efficacy. Excipients may include EDTA as chelating agent or antioxidants such as, e.g., alpha-tocopherol, BHT, BHA and any other excipients commonly used in the preparation of parenteral formulations for intravenous administration. Antioxidants may be selected from any of those compounds known in the art. The amount of other suitable excipients employed can be determined using only routine experimentation.

The resultant parenteral formulation may be maintained under an inert atmosphere and is transferred to suitable containers, e.g. by a cannular system also under the inert atmosphere. Solvents other than water, when required, and other reagents may be chosen from medical grade reagents and solvents well known in the art. Parenteral formulations according to the invention may be packaged in containers. Containers may be chosen which are made of material. Glass containers may be used although it is preferred to use plastic containers, e.g. plastic infusion bags. In one embodiment of the present invention, there is provided a single dosage form suitable for parenteral such as e.g. intravenous administration comprising an effective amount of the benzodiazepine RSV inhibitor compound and a beta-cyclodextrin such as, e.g., HPbCD, SBEbCD or MbCD and, optionally, further excipients commonly used in pharmaceutical compositions as e.g. described hereinabove. The single dosage form contains at least 0.5 mg/ml, preferably at least 1 mg/ml more preferably at least 2mg/ml of the benzodiazepine RSV inhibitor compound. In a single dose, conveniently, 10mg, 20mg, 25mg, 50mg, 75mg, 100mg, 125mg, 150mg, 175mg, 200mg or 250mg are administered to a patent in need.

In another aspect of the invention there is provided a process of preparing a parenteral formulation according to the present invention. The process comprises the step of adding or admixing an aqueous solution, e.g. a isotonic solution, to a benzodiazepine RSV inhibitor compound and a beta-cyclodextrin and optionally other pharmaceutically acceptable excipients such as e.g. an antioxidant in a suitable vessel from a material which is non-reactive or substantially non-reactive with the parenteral formulation.

In one aspect of the present invention, the galenic formulation is lyophilized. Lyophilization, or more commonly known as "freeze-drying", is a process which extracts water from a solution to form a granular solid or powder. The process is carried out by freezing the solution and subsequently extracting any water or moisture by sublimation under vacuum. Lyophilization is particularly useful for developing pharmaceutical drug products that are reconstituted and administered to a patient by injection, for example parenteral drug products. In accordance with one embodiment of the present invention, the benzodiazepine RSV inhibitor compound is lyophilized in the presence of a bulking agent and/or a non-volatile co-solvent to a pharmaceutically acceptable cake.

As used herein, the term "bulking agent" refers to an ingredient that provides bulk to the pharmaceutical composition. Examples of bulking agents include, without limitation, mannitol, trehalose, lactose, sucrose, polyvinyl pyrrolidone, sucrose, glycine, cyclodextrins, dextran, solid PEGs and derivatives and mixtures thereof. Particularly useful as bulking agents in accordance with the present invention are beta-cyclodextrins, e.g., HPbCD, MbCD or SBEbCD.

As will be appreciated by the skilled person, some components of the formulation can serve more than one function. Mannitol for instance may be used as isotonic agent, but may also act as bulking agent for lyophilization.

As used herein, a nonvolatile cosolvent refers to a substance having a vapor pressure lower than 0.50 mm Hg at 25°C. The purpose of the nonvolatile cosolvent is to facilitate the dissolution of a poorly water-soluble therapeutic compound in water in order to form a solution. Examples of a nonvolatile cosolvent include, without limitation, alkylene glycols such as, liquid PEG MW200-800, propylene glycol, polyhydric alcohols, e.g., mannitol, sorbitol and xylitol; polyoxyethylenes; linear polyols, e.g., ethylene glycol, 1,6-hexanediol, neopentyl glycol and methoxypolyethylene glycol; and mixtures thereof.

Surfactants can also be optionally used in the pharmaceutical composition. Examples of surfactants include fatty acid and alkyl sulfonates; benzethanium chloride, e.g., HYAMINE 1622 from Lonza, Inc. (Fairlawn, NJ); polyoxyethylene sorbitan fatty acid esters, e.g., the TWEEN Series from Uniqema (Wilmington, DE); and natural surfactants, such as sodium taurocholic acid, 1-palmitoyl-2-Sn-glycero-3-phosphocholine, lecithin and other phospholipids. Such surfactants, e.g., minimize aggregation of lyophilized particles during reconstitution of the product. These surfactants may comprise from about 0.001% to about 5% w/v.

In order to prepare a suitable pharmaceutically acceptable lyophilized cake of the benzodiazepine RSV inhibitor compounds, an appropriate amount, e.g., conveniently a therapeutically effective amount of the benzodiazepine RSV inhibitor compound is mixed with a water or an aqueous-based solvent, a nonvolatile cosolvent (optional) and a bulking agent to form a solution. The solution contains, e.g., a concentration of the bulking agent from about 1% to about 60% (w/v), e.g., 20% to about 50% or 30 to 50%, e.g. 40%. A preferred bulking agent in accordance with the present invention is a beta-cyclodextrin, such as e.g. HPbCD, SBEbCD or MbCD. Furthermore, the solution optionally contains, e.g., a concentration of the nonvolatile cosolvent from about 0.01% to about 30% (w/v), e.g., about 0.1% to about 20%, e.g., about 1% to about 10%. Optionally, a surfactant can also be added. The resulting solution is typically homogeneous and optically clear. The solution does not comprise any solvents having a relatively high vapor pressure, e.g. lower alcohols, such as ethanol, isopropanol or tert-butanol. The concentration of the benzodiazepine RSV inhibitor compound in the solution is preferably at least 0.1 mg/ml, preferably at least 0.5 mg/ml, more preferably at least 1 mg/ml. Typically, the concentration of the benzodiazepine RSV inhibitor compound is between 1 mg/ml and 50 mg/ml, e.g. between 2 mg/ml and 8 mg/ml. In another preferred embodiment, the solution before lyophilization contains 1 mg/ml to 10 mg/ml such as, e.g., 4 mg/ml to 7 mg/ml of benzodiazepine RSV inhibitor compound (free base equivalent) in 10% to 60% such as, e.g., 30% to 50% beta-cyclodextrin. Though there may be additional excipients such as e.g. nonvolatile cosolvent or a surfactant present, in one preferred embodiment, the solution does not contain additional excipients.

Once mixed, the solution is filled into a container that is suitable for lyophilization, e.g., a glass vial. The lyophilization cycle typically includes the following steps: a freezing step, a primary drying step and a secondary drying step. In the freezing step, the solution is cooled. The temperature and duration of the freezing step is chosen such that all of the ingredients in the composition are completely frozen. For example, a suitable freezing temperature is approximately -40°C. The water in the formulation becomes crystalline ice. The balance of the formulation in the frozen state may be crystalline, amorphous or a combination thereof. In the primary drying step, the ice formed during freezing is removed by sublimation at subambient temperatures (although greater than the freezing temperature) under vacuum. For example, the chamber pressure used for sublimation can be from about 40 milliTorr to 400 milliTorr and the temperature be between -30°C to -5°C. During the primary drying step, the formulation should be maintained in the solid state below the collapse temperature ("T_{c}") of the formulation. The T_{c} is the temperature above which the freeze-dried cake loses macroscopic structure and collapses during freeze-drying. For amorphous products the glass transition temperature ("T_{g}") or for crystalline products the eutectic temperature ("Tₑ") are approximately the same as T_{c}. In addition, the T_{g} for the maximally freeze concentrated solution ("T'_{g}") is important to the development of lyophilization cycles because this represents the highest temperature that is safe for the composition for primary drying. After primary drying, any residual amounts of liquid which could not be removed by sublimation is removed by secondary drying, i.e., desorption. The temperature during secondary drying is near or greater than ambient temperature. After lyophilization, the pharmaceutical composition becomes a cake. Such a cake should be pharmaceutically acceptable. As used herein, a "pharmaceutically acceptable cake" refers to a non-collapsed solid drug product remaining after lyophilization that has certain desirable characteristics, e.g. pharmaceutically acceptable, long-term stability, a short reconstitution time, an elegant appearance and maintenance of the characteristics of the original solution upon reconstitution. The pharmaceutically acceptable cake can be solid, powder or granular material. The pharmaceutically acceptable cake may also contain up to five percent water by weight of the cake.

During the lyophilization process, neither the nonvolatile cosolvent nor bulking agent will sublime from the pharmaceutical composition. In the final pharmaceutically acceptable cake, the cake, e.g. comprises from about 0% to about 90% (w/w) of nonvolatile cosolvent; e.g., from about 5% to about 80% (w/w); e.g., from about 10% to about 70%; e.g., from about 20% to about 60% (w/w). Furthermore, the cake, e.g., comprises from about 10% to about 99% (w/w) of the bulking agent; e.g., from about 20% to about 70% (w/w); e.g., from about 30% to about 60% (w/w). Where the bulking agent is a beta-cyclodextrin, the cake conveniently comprises of the beta-cyclodextrin from about 50% to about 99.5% (w/w), e.g., from about 80% to about 99.5% (w/w), e.g., from about 95% to about 99% (w/w). The pharmaceutical composition or pharmaceutically acceptable cake will suitably contain between 0.1 mg and 100 mg of the therapeutic compound per unit dose, e.g., 0.1 mg, 1 mg, 5 mg, 10 mg, 20 mg, 25 mg, 50 mg or 100 mg per unit dose. As used herein, a "pharmaceutically acceptable cake" refers to a non-collapsed solid drug product remaining after lyophilization that has certain desirable characteristics, e.g.; pharmaceutically acceptable, long-term stability, a short reconstitution time, an elegant appearance and maintenance of the characteristics of the original solution upon reconstitution. The pharmaceutically acceptable cake can be solid, powder or granular material. The pharmaceutically acceptable cake may also contain up to five percent water by weight of the cake.

The pharmaceutically acceptable cake can be reconstituted, e.g., for instant use. If all required components (e.g., buffer, isotonic agent) are present in the cake, sterile de-ionized water may be used for the reconstitution. Alternatively, an isotonic solution such as e.g. Plasma-Lyte A ® from Baxter or Ringer Acetate Solution from Baxter may be used for reconstitution. The reconstituted solution typically contains about 0.5 mg/ml to about 10 mg/ml such as, e.g., 1 mg/ml to 4 mg/ml of benzodiazepine RSV inhibitor compound (free base equivalent). Preferably, the reconstituted solution contains at least 0.5 mg/ml, at least 1 mg/ml or at least 2 mg/ml of benzodiazepine RSV inhibitor compound. The reconstituted solution typically contains 5% to 50%, e.g., 10% to 40%, e.g., 20% to 30% of a beta-cyclodextrin, such as e.g. HPbCD, MbCD or SBEbCD. The pH of the reconstituted solution is preferably between 4 to 8, e.g., 4.5 to 7, e.g. about 5.

The formulations of the present invention are useful for treating viral infections. In a preferred embodiment the infection is viral infection disclosed in WO2004/026843, in particular a RSV or an influenza virus, a metapneumovirus, measles, parainfluenza or mumps virus. Accordingly the present invention provides a method of treating a viral infection, in particular a RSV infection, in a patient in need, and in particular in an infant or small child having a RSV infection, comprising parenterally (e.g. intravenously) administering a formulation comprising an effective amount of a benzodiazepine RSV inhibitor compound in a reconstituted solution comprising 5 to 50%, preferably 10 to 40% or 20% to 30% of a beta-cyclodextrin (e.g. HPbCD or SBEbCD).

In another embodiment, there is provided use of a pharmaceutical composition suitable for parenteral administration comprising a benzodiazepine RSV inhibitor compound and a beta-cyclodextrin, preferably HPbCD, for the manufacture of a medicament for the treatment of a viral infection and in particular a RSV infection. In a preferred embodiment, the present invention provides a pediatric medicament, i.e. a medicament useful for the treatment of infants or small children having e.g. a RSV infection.

The following examples are intended to illustrate the invention and are not to be construed as being limitations thereto.

### Example 1

6 mg/ml (S)-1-(2-Fluoro-phenyl)-3-(2-oxo-5-phenyl-2,3-dihydro-1H-benzo[e][1,4]diazepin-3-yl)-urea (free base equivalent) is dissolved in 40% HPbCD as follows:
a. In a clean compounding vessel, charge WFI at 90% of the calculated amount needed.
b. Slowly charge cyclodextrin in a), mix with a propeller stirrer until all the cyclodextrin is dissolved.
c. Slowly charge (S)-1-(2-Fluoro-phenyl)-3-(2-oxo-5-phenyl-2,3-dihydro-1H-benzo[e][1,4]diazepin-3-yl)-urea benzenesulfonate monohydrate in b), mix with a propeller stirrer for at least 1 hour or until a clear solution is obtained.
d. Qs the batch size with the rest of WFI.
e. Filter the solution using 0.22 micron filter
f. Fill the solution in 6R vial at 2.2 ml.
g. Partially insert the lyo rubber stoppers onto the vials.
h. Load the vials into a lyophillizer.
i. Start the lyophilized cycle at by following the steps in Table 1.
j. At the end of the cycle, collapse the shelf to fully close the stoppers
k. Unload the vials for capping.

With overfill to 2.2 ml each vial contains a target of 19.19 mg of (S)-1-(2-Fluoro-phenyl)-3-(2-oxo-5-phenyl-2,3-dihydro-1H-benzo[e][1,4]diazepin-3-yl)-urea benzenesulfonate monohydrate.

**Table 1: Lyophilized cycle**

| **Step** | **Operation** | **Time/[hh:mm]** | **Shelf** **Temperature** | **Chamber** **Pressure** |
|---|---|---|---|---|
| 1 | Vial loading | As required | 20°C | Ambient |
| 2 | Cooling down | 00:15 | 20°C to 5°C | Ambient |
| 3 | 5°C hold | 01:00 | 5°C | Ambient |
| 4 | Freeze ramp | 00:10 | 5°C to -5°C | Ambient |
| 5 | Freeze hold | 01:00 | -5°C | Ambient |
| 6 | Freeze ramp | 00:45 | -5°C to -50°C | Ambient |
| 7 | Freeze hold | Min. 03:00 | -50°C | Ambient |
| | | Max. 70:00 | | |
| 8 | Chamber vacuum | 00:10 | -50°C | 0.145 mbar^{a} |
| 9 | Primary drying ramp | 06:40 | -50°C to -10°C | 0.145 mbar^{a} |
| 10 | Primary drying hold | 25:00 | -10°C | 0.145 mbar^{a} |
| 11 | Secondary drying ramp | 07:30 | -10°C to 35°C | 0.145 mbar^{a} |
| 12 | Secondary drying hold | 04:00 | 35°C | 0.145 mbar^{a} |

| | | | | |
|---|---|---|---|---|
| ^{a} Chamber pressure is controlled using sterile filtered nitrogen. The pressure is determined by capacitance manometers. | | | | |

Obtained is a white or slightly yellowish lyophilizate cake or granules.

### Example 2:

The lypholized cake of Example 1 is reconstituted with 3.8 ml Ringer-Acetate.solution or Plasma-Lyte A ® solution (pH 6-8, commercially available from Baxter) to obtain 4.4 ml of 3 mg/ml (S)-1-(2-Fluoro-phenyl)-3-(2-oxo-5-phenyl-2,3-dihydro-1H-benzo[e][1,4]diazepin-3-yl)-urea in 20%HPbCD. Under aseptic technique, withdraw 3.8 ml of the reconstituted solution (using syringe) and introduce into the vial containing the lyophilized cake. Swirl or shake the vial until all the solid dissolve. The reconstituted solution is clear, colorless to slightly yellowish. The reconstituted solution is ready for intravenous infusion. Reconstituted solution pH is about 5.

### Example 3:

6 mg/ml (S)-1-(2-Fluoro-phenyl)-3-(2-oxo-5-phenyl-2,3-dihydro-1H-benzo[e][1,4]diazepin-3-yl)-urea (free base equivalent) is dissolved in 40% HPbCD, with addition of 15 mM phosphate buffer, pH 7. The lyophilized cake of this solution is reconstituted with 3.8 ml of 5% dextrose solution to obtain 4.4 ml of 3 mg/ml (S)-1-(2-Fluoro-phenyl)-3-(2-oxo-5-phenyl-2,3-dihydro-1H-benzo[e][1,4]diazepin-3-yl)-urea in 20%HPbCD.

## Claims

1. A parenteral formulation comprising a benzodiazepine RSV inhibitor compound, or a pharmaceutically acceptable salt thereof, and a beta-cyclodextrin in an aqueous solution, wherein the RSV inhibitor compound is (S)-1-(2-Fluoro-phenyl)-3-(2-oxo-5-phenyl-2,3-dihydro-1H-benzo[e][1,4]diazepin-3-yl)-urea besylate salt and the beta-cyclodextrin is HPbCD (hydroxypropyl-beta-cyclodextrin), and wherein the formulation comprises between 10% to 50% HPbCD and at least 0.5 mg/ml of the RSV inhibitor compound.

2. The formulation as claimed in claim 1, for the treatment of a Respiratory Syncytial Virus infection.

3. Use of a formulation as claimed in claim 1, for the manufacture of a medicament for the treatment of a Respiratory Syncytial Virus infection.

## Patentansprüche

1. Parenterale Formulierung, umfassend eine Benzodiazepin-RSV-Inhibitorverbindung, oder ein pharmazeutisch akzeptables Salz davon, und ein beta-Cyclodextrin in einer wässrigen Lösung, wobei die RSV-Inhibitorverbindung (S)-1-(2-Fluor-phenyl)-3-(2-oxo-5-phenyl-2,3-dihydro-1H-benzo[e][1,4]diazepin-3-yl)-harnstoffbesylatsalz ist und das beta-Cyclodextrin HPbCD (Hydroxypropyl-beta-cyclodextrin) ist und wobei die Formylierung zwischen 10 % bis 50 % HPbCD und mindestens 0,5 mg/ml der RSV-Inhibitorverbindung umfasst.

2. Formulierung gemäß Anspruch 1 für die Behandlung einer Synzytial-Virusinfektion der Atemwege.

3. Verwendung einer Formulierung gemäß Anspruch 1 für die Herstellung eines Medikaments für die Behandlung einer Synzytial-Virusinfektion der Atemwege.

## Revendications

1. Formulation parentérale comprenant un composé benzodiazépine inhibiteur de VRS ou un sel pharmaceutiquement acceptable de celui-ci, et une bêta-cyclodextrine dans une solution aqueuse, **caractérisée en ce que** le composé inhibiteur de VRS est le sel de bésylate de (S)-1-(2-fluoro-phényl)-3-(2-oxo-5-phényl-2,3-dihydro-1H-benzo[e] [1,4]diazépin-3-yl)-urée et la bêta-cyclodextrine est HPbCD (hydroxypropyl-bêta-cyclodextrine), et où la formulation comprend entre 10 % et 50 % de HPbCD et au moins 0,5 mg/ml du composé inhibiteur de VRS.

2. Formulation selon la revendication 1, pour le traitement d'une infection par le virus respiratoire syncytial.

3. Utilisation d'une formulation selon la revendication 1, pour la fabrication d'un médicament pour le traitement d'une infection par le virus respiratoire syncytial.
